(19) Europäisches Patentamt
European Patent Office
Office européen des brevets

(11) **EP 3 747 924 A1**

(12) **EUROPEAN PATENT APPLICATION**

(43) Date of publication:
**09.12.2020  Bulletin 2020/50**

(21) Application number: **19178722.5**

(22) Date of filing: **06.06.2019**

(51) Int Cl.:
**C08G 18/79** (2006.01)     **C08G 18/76** (2006.01)
**C08G 18/18** (2006.01)     **C08G 18/16** (2006.01)
**C08G 18/42** (2006.01)     **C08G 18/44** (2006.01)
**C08G 18/40** (2006.01)     **C08G 18/48** (2006.01)
**C07D 251/34** (2006.01)     **C08G 101/00** (2006.01)
**C08G 18/02** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Covestro Deutschland AG
51373 Leverkusen (DE)**

(72) Inventor: **The designation of the inventor has not
yet been filed**

(74) Representative: **Levpat
c/o Covestro AG
Gebäude 4825
51365 Leverkusen (DE)**

(54) **RIGID POLYURETHANE FOAM**

(57)    The present invention relates to a process for preparing a (poly)isocyanurate, a (poly)isocyanurate prepared with said process and use thereof. The process according to the present invention in which a free radical initiator is used as a trimerization catalyst provides a trimerization catalytic reaction system in which the reaction rate is mild and controllable, and the trimerization reaction of an isocyanate can be carried out in a very moderate manner.

Fig. 1

**Description**

**Technical Field**

The present invention relates to a process for preparing a (poly)isocyanurate, a (poly)isocyanurate prepared with said process and use thereof.

**Background**

The trimerization of isocyanate functional groups is a curing crosslinking reaction commonly used in polyurethane reaction systems. The trimerization reaction is usually carried out by the polymerization of three isocyanate groups into one isocyanurate six-membered ring in the presence of one or more trimerization catalysts. However, the trimeric catalysts usually have relatively high catalytic activity, which makes the trimerization reaction relatively violent.

The trimeric catalytic reaction of isocyanates is a curing crosslinking method commonly used in the field of polyurethane materials. The rigid structure of the isocyanurate six-membered ring formed by the trimerization reaction can provide a high degree of crosslinking to enhance the mechanical strength of the cured polyurethane material, and at the same time, due to the high carbon, nitrogen and oxygen contents of the isocyanurate six-membered ring and the excellent flame retardancy produced by the conjugate aromatic structure can also help to improve the overall flame retardant properties of the polyurethane material. For these reasons, in the field of polyurethane materials, the trimerization of isocyanate functional groups has been widely used.

It is necessary for the trimerization reaction of the isocyanate to take place in the presence of a trimerization catalyst. The trimerization catalyst is generally a phosphorus-containing substance, an amine substance or a metal salt substance, or some suitable combination thereof. The trimerization reactions initiated by these catalysts are usually fast, and these catalysts will catalyze the trimerization reactions to carry out in a short time, for example in several tens of seconds or several minutes. Along with the occurrence of the trimerization reaction, a large amount of reaction heat is released, and since the polyurethane resin is a poor heat conductor, the heat released by these trimerization reactions is difficult to be effectively released into the external environment in a short time, so the heat released by these reactions is retained in the resin, resulting in a markedly rapid increase in the temperature of the entire polyurethane resin reaction system. This increase in temperature, in turn, further accelerates the isocyanate trimerization and/or other polymerizations, thereby generating more heat which in turn continues to cause the temperature of the resin system to rise. This "releasing the heat"-"promoting the temperature rise"-"releasing the heat again"-"promoting the temperature rise again" cycle mode is a self-accelerating catalytic reaction mode that will cause the isocyanate trimerization rate to increase, making its controllability become bad. Especially when the trimerization reaction is required to be carried out slowly in some specific occasions, it is difficult to achieve the target using these conventional trimerization catalysts.

CN101848951A discloses a process for preparing a highly stable, liquid isocyanurate-modified PMDI composition having a higher viscosity and generally equivalent functionality than the conventional PMDI. It is characterized in that it comprises the steps of: (a) tripolymerizing a conventional PMDI having a viscosity of from about 30 to about 300 cps in the presence of a catalytically effective amount of a trimerization catalyst to produce an isocyanurate-containing PMDI having a viscosity in a range of from about 2,000 mPas to about 200,000 mPas at 25°C; (b) passivating the trimerization catalyst with a catalyst passivating agent to provide a mixture containing an isocyanurate-modified PMDI and a passivated trimerization catalyst; and (c) blending the mixture of step (b) with an amount of the conventional PMDI sufficient to provide a blend having a viscosity in the range of from about 400 mPas to about 20,000 mPas at 25°C and a free NCO content comparable to that of the conventional PMDI.

CN108026232A discloses a method for preparing a rigid PUR-PIR foam by reacting a PUR-PIR system being consisted of: 1) an isocyanate component (A) comprising: 1.1) 15-25 wt % of isocyanurate groups; and 1.2) 30-55 wt % of monomeric MDI; 1.3) an NCO content of 23-30 wt% (EN ISO 11909:2007), all based on the total weight of the isocyanate component; and 2) a polyol formulation (B); in the presence of: 3) blowing agents (C); 4) catalysts (D); and optionally 5) further auxiliary and additives (E); wherein the isocyanate component (A) is obtained by a method comprising: (i) preparing an isocyanate blend (A2) by partially trimerizing an isocyanate mixture (A1) comprising oligomeric and monomeric MDI, wherein (A1) prior to trimerization comprises: a total monomeric MDI content of 55-80 wt % based on the total weight of (A1); and a viscosity of <30 mPas at 25°C (DIN EN ISO 3219:1994); and optionally (ii) mixing the partially trimerized isocyanate blend A2 obtained in (i) with further isocyanates (A3), preferably polymeric MDI; and wherein the system comprising the components (A) to (E) has an isocyanate index of 90 to 150.

Despite the above disclosure, there is an urgent need in the industry for a mild and controllable reaction process for the preparation of (poly)isocyanurate.

## Summary of the Invention

In an aspect, the present invention provides a process for preparing a (poly)isocyanurate, which is produced by reaction of a reaction system comprising the following components:

one or more polyisocyanates;
at least one free radical initiator.

Preferably, said isocyanate is selected from a group consisting of an aromatic isocyanate, preferably diphenylmethane diisocyanate, toluene diisocyanate, diphenylmethane diisocyanate or a combination thereof, particularly preferably diphenylmethane diisocyanate or (poly)diphenylmethane diisocyanate.

Preferably, the content of said free radical initiator is 0.1-5 pbw, preferably 0.2-4 pbw, more preferably 0.4-3 pbw, based on the total weight of said reaction system.

Preferably, said free radical initiator is selected from a group consisting of a peroxide and/or an azo compound.

Preferably, said peroxide is selected from a group consisting of ketone peroxide, carbonate peroxide, acyl peroxide, ester peroxide, hydrogen peroxide, alkyl peroxide or any combination thereof, preferably tert-butyl benzoate peroxide, benzoyl peroxide or any combination thereof.

Preferably, said azo compound is selected from a group consisting of azobisisobutyronitrile, azobisisoheptonitrile or any combination thereof.

Preferably, the reaction time of said reaction system >5 hours, preferably >10 hours, particularly preferably >24 hours.

Preferably, the difference between the temperatures after and before the reaction of said reaction system $< \pm 2°C$.

Through repeated experiments, we have unexpectedly discovered that free radical initiators can catalyze the trimerization of isocyanates. Moreover, free radical initiators can provide a smooth and controllable polymerization compared to conventional trimerization catalysts.

The method for producing (poly)isocyanurate of the present invention changes the state in which the reaction strength of the trimerization reaction in the prior art is severe and the temperature rises a lot. When the isocyanate reaction system of the present invention experiences the trimerization reaction, there can be sufficient time for the heat released by the chemical reaction to be diffused into the external environment, rather than retained in the resin system. Therefore, this part of the heat does not raise the temperature of the entire resin system, so that no additional temperature change occurs while the resin undergoes a trimerization reaction, and the temperature of the entire resin is kept constant at a predetermined value, so that the chemical reaction of the resin system has the better controllability.

In still another aspect of the present invention, there is provided a (poly)isocyanurate which is produced by the aforementioned process for preparing the (poly)isocyanurate of the present invention.

In a further aspect of the invention, there is provided the use of a free radical initiator for catalyzing the trimerization of isocyanate to prepare the (poly)isocyanurate. The reaction system for the trimerization reaction includes the following components:

one or more polyisocyanates;

at least one free radical initiator.

Preferably, said free radical initiator is selected from a group consisting of a peroxide and/or an azo compound.

Preferably, said peroxide is selected from a group consisting of ketone peroxide, carbonate peroxide, acyl peroxide, ester peroxide, hydrogen peroxide, alkyl peroxide or any combination thereof, preferably tert-butyl benzoate peroxide, benzoyl peroxide or any combination thereof.

Preferably, said azo compound is selected from a group consisting of azobisisobutyronitrile, azobisisoheptonitrile or any combination thereof.

Preferably, the content of said free radical initiator is 0.1-5 pbw, preferably 0.2-4 pbw, more preferably 0.4-3 pbw, based on the total weight of said reaction system.

In a further aspect of the invention, there is provided the use of (poly)isocyanurate prepared by the process for the preparation of (poly)isocyanurate of the present invention for heat-insulation.

In still another aspect of the present invention, there is provided a reaction system for preparing a polyurethane-(poly)isocyanurate compound, comprising:

the (poly)isocyanurate prepared by the aforementioned process of the present invention;

at least one polyol; and

optionally, at least one foaming agent.

The foaming agent that can be used in the present invention is preferably 0.10-3.50wt%, preferably 0.5-2.8wt%, particularly preferably 1.5-2.6wt% of water, based on the total weight of the polyol.

Preferably, the reaction system of the present invention may further comprise at least one catalyst. Catalysts useful in the present invention preferably include a foaming catalyst, a gelling catalyst, and a trimerization catalyst. Among others, the foaming catalyst is preferably one, or any mixture of two or more than two selected from a group consisting of pentamethyldiethylenetriamine, bis-dimethylaminoethyl ether, N,N,N',N'-tetramethylethylenediamine, N,N,N',N'-tetramethylbutanediamine and tetramethylhexamethylene diamine; the gelling catalyst is preferably one or any mixture selected from a group consisting of dimethylcyclohexylamine and dimethyl benzylamine; the trimerization catalyst is preferably one, or any mixture of two or more than two selected from a group consisting of methyl ammonium salt, ethyl ammonium salt, octyl amine salt or hexahydrotriazine and organometallic base. The content of the catalyst is preferably 0.50-4.00 pbw, based on the total weight of the polyol component.

The reaction system of the present invention preferably further comprises a surfactant. The surfactant, preferably silicone oil, is present in an amount of 0.1-5.0wt%, preferably 0.5-4.0wt%, particularly preferably 1.5-3.0wt%, based on the total weight of the polyol component.

In still another aspect of the present invention, there is provided a polyurethane-(poly)isocyanurate compound produced from the reaction system for producing the polyurethane-(poly)isocyanurate compound of the present invention.

In still another aspect of the present invention, there is a heat-insulation material, comprising the (poly)isocyanurate or the polyurethane-(poly)isocyanurate compound produced by the process for preparing the (poly)isocyanurate of the present invention.

Optionally, said heat-insulation material is selected from a group consisting of a thermal insulation board, a refrigerator wall, a cold-insulation tube shell, a refrigerated truck compartment side panel and a car-carried refrigerator wall.

## Brief Description of the Drawings

The present invention will be exemplarily described below with reference to the accompanying drawings:

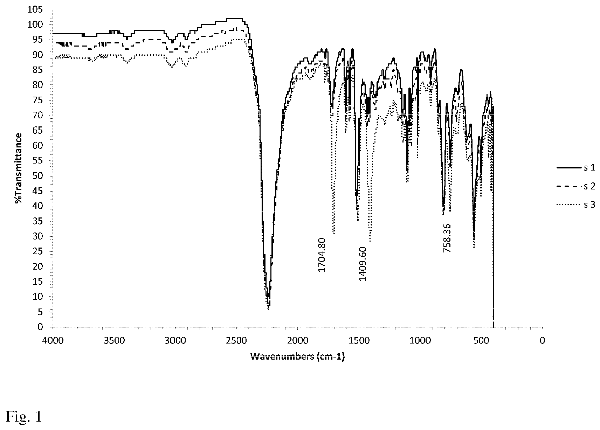

Figure 1 shows an infrared spectrum of the example and the comparative example of the present invention, wherein the curve s1 in Fig. 1 is the curve for the system only having the isocyanate 44V20; the curve s2 in Fig. 1 is the curve for the system at the initial stage, in which 2wt% TBPB is added to the isocyanate 44V20, wherein the viscosity has not changed yet; and the curve s3 in Fig. 1 is the curve for the system after 56 days, in which 2wt% TBPB is added to the isocyanate 44V20.

## Detailed Description of the Invention

The following terms used in the present invention have the following definitions or explanations: pbw refers to the mass fraction of each component of the reaction system;

The functionality refers to a value determined according to the industry formula:

Functionality = hydroxyl value * molecular weight / 56100; wherein the molecular weight is determined by GPC high performance liquid chromatography;

The isocyanate index refers to the value calculated by the following formula:

$$\text{Isocyanate index (\%)} = \frac{\text{Mole number of isocyanate groups (NCO groups) in component A}}{\text{Mole number of isocyanate group-reactive groups in component B}} \times 100\%$$

.

In the first aspect, the present invention provides a process for preparing a (poly)isocyanurate, which is produced by reaction of a reaction system comprising the following components:

one or more polyisocyanates;

at least one free radical initiator.

Any organic polyisocyanate can be used in the above preparation process of the present invention, including aromatic, aliphatic and alicyclic polyisocyanates and combinations thereof. The polyisocyanate can be represented by the formula R(NCO)$_n$, wherein R represents an aliphatic hydrocarbon group having 2 to 18 carbon atoms, an aromatic hydrocarbon group having 6 to 15 carbon atoms, and an araliphatic hydrocarbon group having 8 to 15 carbon atoms, n=2-4.

The useful polyisocyanate preferably includes, but is not limited to ethylene diisocyanate, tetramethylene 1,4-diisocyanate, hexamethylene diisocyanate (HDI), dodecylene 1,2-diisocyanate, cyclobutane-1,3-diisocyanate, cyclohexane-1,3-diisocyanate, cyclohexane-1,4-diisocyanate, 1-isocyanato-3,3,5-trimethyl-5-isocyanatomethylcyclohexane, hexahydrotoluene-2,4-diisocyanate, hexahydrophenyl-1,3-diisocyanate, hexahydrophenyl-1,4-diisocyanate, perhydrogenated-diphenylmethane 2,4-diisocyanate, perhydrogenated diphenylmethane 4,4-diisocyanate, phenylene 1,3-diisocyanate, phenylene 1,4-diisocyanate, stilbene-1,4-diisocyanate, 3,3-dimethyl-4,4-diphenyl diisocyanate, toluene-2,4-diisocyanate (TDI), toluene-2,6-diisocyanate (TDI), diphenylmethane-2,4'-diisocyanate (MDI), diphenylmethane-2,2'-diisocyanate (MDI), diphenylmethane-4,4'-diisocyanate (MDI), mixtures of diphenylmethane diisocyanate and/or diphenylmethane diisocyanate homologs having more rings, polyphenylene polymethylene polyisocyanate (polymerized MDI), naphthalene-1,5-diisocyanate (NDI), their isomers, any mixtures of them and their isomers.

Useful polyisocyanates further include isocyanates modified with carbodiimides, allophanates or isocyanates, preferably but not limited to diphenylmethane diisocyanate, carbodiimide modified diphenylmethane diisocyanate, their isomers, any mixtures of them and their isomers.

When used in the present invention, polyisocyanates include isocyanate dimers, trimers, tetramers, or combinations thereof.

In certain embodiments of the invention, the isocyanate is selected from a group consisting of aromatic isocyanates, preferably diphenylmethane diisocyanate, toluene diisocyanate, diphenylmethane diisocyanate or combinations thereof, particularly preferably diphenylmethane diisocyanate or (poly)diphenylmethane diisocyanate.

In a preferred embodiment of the invention, the polyisocyanate component is selected from a group consisting of poly-MDIs.

The organic polyisocyanate of the present invention has an NCO content of 20-33wt%, preferably 25-32wt%, particularly preferably 30-32wt%. The NCO content is measured by GB/T 12009.4-2016.

Organic polyisocyanates can also be used in the form of polyisocyanate prepolymers. These polyisocyanate prepolymers can be obtained by reacting an excess of the above organic polyisocyanate with a compound having at least two isocyanate-reactive groups at a temperature of, for example, 30-100°C, preferably about 80°C. The polyisocyanate prepolymer of the present invention has an NCO content of 20-33 wt%, preferably 25-32 wt%. The NCO content is measured by GB/T 12009.4-2016.

The free radical initiator refers to an agent capable of generating a free radical in a free radical reaction. It can be also known as a free radical initiator. The process of generating free radicals is called a chain initiation. Free radical initiators that can be used in the present invention include but are not limited to peroxy compound initiators and azo initiators and redox initiators and the like, and the peroxide initiators are further classified into organic peroxide initiators and inorganic peroxide initiators.

The organic peroxy compound has a structural formula of R-O-O-H or R-O-O-R, wherein R is an alkyl group, an acyl group, a carbonate group or the like. It further comprises: an acyl peroxide such as benzoyl peroxide and lauroyl peroxide; a hydroperoxide such as cumene hydroperoxide, t-butyl hydroperoxide; a dialkyl peroxide such as di-tert-butyl peroxide, dicumyl peroxide; an ester peroxide such as tert-butyl peroxybenzoate, tert-butyl peroxy-t-pentanoate; a ketone peroxide such as methyl ethyl ketone peroxide, cyclohexanone peroxide; a dicarbonate peroxide such as diisopropyl peroxy dicarbonate, dicyclohexyl peroxy dicarbonate. In general, the activity order of the organic peroxides is: dicarbonate peroxide > acyl peroxide > ester peroxide > dialkyl peroxide > hydroperoxide.

The azo initiators include azobisisobutyronitrile and azobisisoheptonitrile, which are low activity initiators. The commonly used one is azobisisobutyronitrile, which is used in a temperature range of 50-65 °C, and is uniformly decomposed, only forming one free radical without other side reactions. It is relatively stable, and it can be stored safely in a pure state, but it is also rapidly decomposed at 80-90 °C. Its disadvantage is that the decomposition rate is low, and the formed isobutyronitrile free radical lacks the dehydrogenation ability, so it cannot be used as an initiator for graft polymerization.

In general, azobisisoheptonitrile has a higher activity and a high initiating efficiency, and therefore can replace azobisisobutyronitrile. Dimethyl azobisisobutyrate (AIBME) has the following characteristics: moderate initiating activity, easy control of polymerization, no residue in the polymerization process, high product conversion rate and harmless decomposition products, and therefore is the best substitute for azobisisobutyronitrile (AIBN). The peroxide initiator and the azo initiator have a higher decomposition temperature (50-100°C), which limits the application in the low-temperature polymerization.

In an embodiment of the invention, the free radical initiator is present in an amount of 0.1-5 pbw, preferably 0.2-4 pbw, more preferably 0.4-3 pbw, based on the total weight of the reaction system.

Preferably, the free radical initiator is selected from a group consisting of peroxides and/or azo compounds.

Preferably, said peroxide is selected from a group consisting of ketone peroxide, carbonate peroxide, acyl peroxide, ester peroxide, hydrogen peroxide, alkyl peroxide or any combination thereof, more preferably tert-butyl benzoate peroxide, benzoyl peroxide or any combination thereof.

Preferably, the azo compound is selected from a group consisting of azobisisobutyronitrile, azobisisoheptonitrile or any combination thereof.

Preferably, the reaction system has a reaction time of >5 hours, preferably >10 hours, particularly preferably >24 hours.

Preferably, the difference between the temperatures after and before the reaction of said reaction system $< \pm 2°C$.

Through repeated experiments, we have surprisingly found that free radical initiators can catalyze the trimerization of isocyanates. Many conventional free radical initiators can be slowly decomposed by themselves at room temperature or under heating, and the radicals obtained from decomposion can continue to catalyze the trimerization of isocyanates. The decomposition rate of these free radical initiators is very slow and can be considered to be carried out in a controlled manner, so that the trimerization catalyzed by it also occurs in a slow manner.

It can be seen that the trimerization reaction of isocyanate can have good controllability by the method provided by the present invention. Accordingly, the present invention provides a mild and controllable isocyanate trimerization process, wherein when the isocyanate system experiences the trimerization reaction, there can be sufficient time for the heat released by the chemical reaction to be diffused into the external environment, rather than retained in the resin system. Therefore, this part of the heat does not raise the temperature of the entire resin system, no additional temperature change occurs while the resin undergoes a trimerization reaction, and the temperature of the entire resin is kept constant at a predetermined value, so that the chemical reaction of the resin system has the better controllability.

In still another aspect of the present invention, there is provided a (poly)isocyanurate which is produced by the aforementioned process for preparing the (poly)isocyanurate of the present invention.

In a further aspect of the invention, there is provided the use of a free radical initiator for catalyzing the trimerization of isocyanate to prepare the (poly)isocyanurate. The reaction system of the trimerization reaction comprises the following components:

one or more polyisocyanates;

at least one free radical initiator.

Preferably, the content of said free radical initiator is 0.1-5 pbw, preferably 0.2-4 pbw, more preferably 0.4-3 pbw, based on the total weight of said reaction system.

Preferably, said free radical initiator is selected from a group consisting of a peroxide and/or an azo compound.

Preferably, said peroxide is selected from a group consisting of ketone peroxide, carbonate peroxide, acyl peroxide, ester peroxide, hydrogen peroxide, alkyl peroxide or a combination thereof, more preferably tert-butyl benzoate peroxide, benzoyl peroxide or any combination thereof.

Preferably, said azo compound is selected from a group consisting of azobisisobutyronitrile, azobisisoheptonitrile or a combination thereof.

In a further aspect of the invention, there is provided the use of (poly)isocyanurate prepared by the process for the preparation of (poly)isocyanurate of the present invention for heat-insulation.

In still another aspect of the present invention, there is provided a reaction system for preparing a polyurethane-(poly)isocyanurate compound, comprising:

the (poly)isocyanurate prepared by the process of the present invention;

at least one polyol; and

optionally, at least one foaming agent.

The reaction system for producing a polyurethane-(poly)isocyanurate compound of the present invention may further comprise one or more polyisocyanates. Useful polyisocyanates are as previously described.

The polyol of the present invention may be a polyether polyol, a polyester polyol, a polycarbonate polyol, and/or a mixture thereof.

The polyol of the present invention is preferably one or more polyether polyols, wherein at least one of the polyether polyols is an amine-initiated polyol. The polyether polyol has a functionality of 2-8, preferably 3-6, and a hydroxyl number of 50-1200, preferably 200-800.

Examples of polyether polyols which can be used in the present invention are aromatic amine-initiated polyether polyols, preferably diphenylmethanediamine-initiated polyether polyols based on propylene oxide.

In an embodiment of the invention, a part of the polyether polyols are selected from a group consisting of sucrose, sorbitol-initiated polyether polyols, and more preferably, a part of the polyether polyol is selected from a group consisting of sucrose, sorbitol-initiated polyether polyols based on propylene oxide.

Said polyester polyol is prepared from the reaction of a dicarboxylic acid or a dicarboxylic anhydride and a polyol. Said dicarboxylic acid is preferably but not limited to an aliphatic carboxylic acid containing 2-12 carbon atoms, for example, butanedioic acid, propanedioic acid, pentanedioic acid, hexanedioic acid, octanedioic acid, nonanedioic acid, decanedioic acid, dodecylcarboxylic acid, cis-butenedioic acid, trans-butenedioic acid, phthalic acid, isophthalic acid, terephthalic acid, or a mixture thereof. Said dicarboxylic anhydride is preferably but not limited to phthalic anhydride, tetrachlorophthalic anhydride, maleic anhydride, a mixture thereof. Said polyol is preferably but not limited to ethylene glycol, diethylene glycol, 1,2-propylene glycol, 1,3-propylene glycol, dipropylene glycol, 1,3-methylpropylene glycol, 1,4-butylene glycol, 1,5-pentylene glycol, 1,6-hexanylene glycol, neo-pentylene glycol, 1,10-decylene glycol, propanetriol, trimethylolpropane or a mixture thereof. Said polyester polyol further comprises a polyester polyol prepared from a lactone. Said polyester polyol prepared from a lactone is preferably but not limited to a polyester polyol prepared from ε-caprolactone.

Said polycarbonate polyol is preferably but not limited to a polycarbonate diol. Said polycarbonate diol can be prepared by reacting a diol with a dihydrocarbyl or diaryl carbonate or phosgene. Said diol is preferably but not limited to 1,2-propylene glycol, 1,3-propylene glycol, 1,4-butylene glycol, 1,5-pentylene glycol, 1,6-hexanylene glycol, diethylene glycol, trioxane diol or a mixture thereof. Said dihydrocarbyl or diaryl carbonate is preferably but not limited to diphenyl carbonate.

The foaming agent of the present invention may be selected from various physical foaming agents or chemical foaming agents.

Useful foaming agents include water, halogenated hydrocarbons, hydrocarbon compounds, and the like. Useful halogenated hydrocarbons are preferably pentafluorobutane, pentafluoropropane, monochlorotrifluoropropene, hexafluorobutene, HCFC-141b (monofluorodichloroethane), HFC-365mfc (pentafluorobutane), HFC-245fa (pentafluoropropane) or any mixture thereof. Useful hydrocarbon compounds preferably include butane, pentane, cyclopentane (CP), hexane, cyclohexane, heptane, and any mixtures thereof.

The reaction system for producing a polyurethane-(poly)isocyanurate compound of the present invention may further include a catalyst. Catalysts useful in the present invention preferably include a foaming catalyst, a gelling catalyst, and a trimerization catalyst. Among others, the foaming catalyst is preferably one, or any mixture of two or more than two selected from a group consisting of pentamethyldiethylenetriamine, bis-dimethylaminoethyl ether, N,N,N',N'-tetramethylethylenediamine, N,N,N',N'-tetramethylbutanediamine and tetramethylhexamethylene diamine; the gelling catalyst is preferably one or any mixture selected from a group consisting of dimethylcyclohexylamine and dimethyl benzylamine; the trimerization catalyst is preferably one, or any mixture of two or more than two selected from a group consisting of methyl ammonium salt, ethyl ammonium salt, octyl amine salt or hexahydrotriazine and organometallic base. The content of the catalyst is preferably 0.50-4.00 pbw, based on the total weight of the polyol component.

The reaction system for producing a polyurethane-(poly)isocyanurate compound of the present invention may further include a surfactant. The surfactant, preferably silicone oil, is present in an amount of 0.1-5.0wt%, preferably 0.5-4.0wt%, particularly preferably 1.5-3.0wt%, based on the total weight of the polyol component.

In still another aspect of the present invention, there is provided a polyurethane-(poly)isocyanurate compound produced from the reaction system for producing the polyurethane-(poly)isocyanurate compound of the present invention.

In still another aspect of the present invention, there is a heat-insulation material, comprising the (poly)isocyanurate or the polyurethane-(poly)isocyanurate compound produced by the process for preparing the (poly)isocyanurate of the present invention.

Preferably, the heat-insulation material of the present invention is selected from a group consisting of a thermal insulation board, a refrigerator wall, a cold-insulation tube shell, a refrigerated truck compartment side panel and a car-carried refrigerator wall.

## Examples

Raw materials:

Isocyanate 44V20: NCO%: 30.5-32.5%, viscosity: 160-240mP.s@25°C, purchased from Covestro Polymers (China) Co., Ltd.;

tert-butyl benzoate peroxide (TBPB): purchased from purchased from Akzo Nobel;

benzoyl peroxide (BPO): purchased from Sigma-Aldrich;

azobisisobutyronitrile (AIBN): purchased from Sigma-Aldrich;

DMP30: a trimerization catalyst, purchased from Air Products and Chemicals, Inc.;

TMR-2: a trimerization catalyst, purchased from Air Products and Chemicals, Inc.;

PC-41: a trimerization catalyst, purchased from Air Products and Chemicals, Inc.;

K15: a trimerization catalyst, purchased from Air Products and Chemicals, Inc.;

1792: a trimerization catalyst, purchased from Air Products and Chemicals, Inc.;

GTS-THP Gelling Time Meter: purchased from Shanghai SenLan Scientific Instrument Co., Ltd..

The test methods of the examples are as follows:

Gelling time: refers to the time period when the components of the reaction system start to mix until the viscosity reaches a certain value (for example, about 10000 mPa·s). The gelling time of the present invention is the time tested using a gelling time meter. The specific test method is as follows: the components of the reaction system are uniformly mixed and placed in a gelling time meter, and the time period when the button is turned on until the gelling time meter stops working is recorded, which is the gelling time of the present invention.

Viscosity test: Tested according to GB/T 12008.8-1992 Standard.

Comparative Examples 1-5 (Trimerization of isocyanates under conventional trimerization catalysts)

100 parts by weight of the liquid isocyanate raw material 44V20 was placed in a plastic cup, 2 parts by weight of the catalyst was further added, and the mixture was quickly stirred uniformly, and then the changes in viscosity and temperature were monitored. The experimental results were shown in Table 1.

Table 1. Experimental Results of Comparative Examples 1-5

|  | Comparative Examples | | | | |
| --- | --- | --- | --- | --- | --- |
|  | 1 | 2 | 3 | 4 | 5 |
| Catalyst | DMP30 | TMR-2 | PC-41 | K15 | 1792 |
| Temperature upon starting (°C) | 25 | 25 | 25 | 25 | 25 |
| Gelling Time (minutes) | 2 | 0.1 | 5 | 15 | 25 |
| Temperature upon gelling (°C) | 100 | 130 | 110 | 100 | 80 |

It could be seen from the data in Table 1 that for the reaction system with a conventional catalyst, the temperature of the reaction system increased rapidly, the reaction time was very short, and it was difficult to control the reaction state.

Example 1

100 parts by weight of liquid isocyanate raw material 44V20 was placed in a plastic cup, 2 parts by weight of TBPB was added, and the mixture was quickly stirred uniformly, and then placed in an oven at 50°C to monitor the changes in viscosity and temperature. At intervals, samples were taken out and cooled to room temperature (25°C) to test for the viscosity and the NCO content. The test results were shown in Table 2.

Table 2. Test Results of Example 1

| Time (day) | 0 | 7 | 21 | 35 | 49 | 56 |
| --- | --- | --- | --- | --- | --- | --- |
| Temperature upon starting (°C) | 50 | 50 | 50 | 50 | 50 | 50 |
| NCO value | 30.21 | 30.13 | 30.1 | 28.19 | 26.33 | 25.7 |
| Viscosity (mPa.S) | 194 | 225 | 250 | 2200 | 17000 | >100000 |
| Real-time temperature of the resin in the oven (°C) | 50 | 50 | 50 | 50 | 50 | 50 |

It could be found from Table 2 that in the case where isocyanate and free radical initiator coexisted, the resin system changed greatly with time: the NCO value was significantly reduced, the viscosity of the system was significantly increased until solidification, which demonstrated the occurrence of the trimerization reaction. It was demonstrated that the free radicals released by the initiator would catalyze the trimerization of the isocyanate groups, that is to say, three NCOs were consumed to form one isocyanurate group. This consumption would lead to a decrease in the NCO value of the resin system. On the other hand, the formation of isocyanurate groups would lead to an increase in the crosslinking degree of the resin system, thereby increasing the viscosity of the system. Therefore, as the time lapsed, the trimerization reaction would further occur, more NCO groups would be consumed, the NCO value of the resin would gradually decrease, and the viscosity would gradually increase. When the crosslinking degree reached a certain level, the viscosity of the resin system would be greatly increased until it became a colloid or a solid.

For comparison, the raw material liquid isocyanate 44V20 (without adding a free radical initiator) was also placed in an oven at 50°C to monitor the changes in viscosity and temperature. At intervals, samples were taken out and cooled to room temperature (25°C) to test for the viscosity and the NCO content. The test results were shown in Table 3.

Table 3. Changes in Isocyanate (for reference)

| Time (day) | 0 | 7 | 21 | 35 | 49 | 56 |
|---|---|---|---|---|---|---|
| NCO value | 30.85 | 30.89 | 30.59 | 30.82 | 30.7 | 30.8 |
| Viscosity (mPa.S) | 213 | 231 | 244 | 259 | 270 | 285 |
| Real-time temperature of the resin in the oven (°C) | 50 | 50 | 50 | 50 | 50 | 50 |

It could be seen from Table 3 that it would remain stable if only the isocyanate itself was present. At the same ambient temperature, the NCO value of the isocyanate itself remained almost constant, and the change in viscosity was small and slow.

The infrared spectra in Figure 1 could also verify these changes. Compared with the isocyanate without the added free radical initiator, the isocyanates with the added free radical initiator were detected to show three characteristic peaks after 56 days ($1704.80cm^{-1}$, $1409.60cm^{-1}$, $758.36cm^{-1}$), which showed the presence of a isocyanurate group and verified that a trimerization reaction occurred in the presence of a free radical to form an isocyanurate group.

(Note: the curve s1 in Fig. 1 was the curve for the system only having the isocyanate 44V20; the curve s2 in Fig. 1 was the curve for the system at the initial stage, in which 2wt% TBPB was added to the isocyanate 44V20, wherein the viscosity had not changed yet; and the curve s3 in Fig. 1 was the curve for the system after 56 days, in which 2wt% TBPB was added to the isocyanate 44V20).

Example 2:

100 parts by weight of liquid isocyanate raw material 44V20 was placed in a plastic cup, 2 parts by weight of BPO was added, and the mixture was quickly stirred uniformly, and then placed in an oven at 60°C to monitor the changes in viscosity and temperature. At intervals, samples were taken out and cooled to room temperature (25°C) to test for the viscosity and the NCO content. The test results were shown in Table 4.

Table 4. Test Results of Example 2

| Time (day) | 0 | 1 | 2 | 3 | 4 | 5 |
|---|---|---|---|---|---|---|
| NCO value | 30.3 | 29.1 | 27.7 | 27.0 | 25.5 | / |
| Viscosity (mPa.S) | 198 | 850 | 5000 | 14300 | >100000 | Soft-gel |
| Real-time temperature of the resin in the oven (°C) | 60 | 60 | 60 | 60 | 60 | 60 |

It could be seen from Table 4 that, similarly to the above results shown in Table 2, a trimerization reaction occurred in the case where the isocyanate and the free radical initiator BPO coexisted. Since the rate at which BPO decomposed to generate free radicals was higher than the decomposition rate of TBPB, BPO could catalyze the occurrence of the trimerization reaction at a faster rate. Correspondingly, the NCO value of the isocyanate decreased more rapidly and the viscosity increased more rapidly, and the resin system reached a solid state in a shorter period of time. However, since the rate at which BPO decomposed to generate free radicals was still slowly and controllable as a whole, so accordingly the trimerization of the isocyanate under its catalysis was still carried out in a controlled manner, and the temperature of the resin system was also kept stable.

Example 3:

100 parts by weight of liquid isocyanate raw material 44V20 was placed in a plastic cup, 2 parts by weight of AIBN was added, and the mixture was quickly stirred uniformly, and then placed in an oven at 60°C to monitor the changes in viscosity and temperature. At intervals, samples were taken out and cooled to room temperature (25°C) to test for the viscosity and the NCO content. The test results were shown in Table 5.

Table 5. Test Results of Example 3

| Time (day) | 0 | 1 | 2 | 3 |
|---|---|---|---|---|
| NCO value | 30.2 | 27.9 | 26.2 | 25.1 |
| Viscosity (mPa.S) | 200 | 2300 | 18000 | >100000 |
| Real-time temperature of the resin in the oven (°C) | 60 | 60 | 60 | 60 |

It could be seen from Table 5 that, similarly to the above results shown in Tables 2 and 3, a trimerization reaction occurred in the case where the isocyanate and the free radical initiator AIBN coexisted. Since the rate at which AIBN decomposed to generate free radicals was higher, it could catalyze the occurrence of the trimerization reaction at a faster rate. Correspondingly, the NCO value of the isocyanate decreased more rapidly and the viscosity increased more rapidly, and the resin system reached a solid state in a shorter period of time. However, since the rate at which AIBN decomposed to generate free radicals was still slowly and controllable as a whole, so accordingly the trimerization of the isocyanate under its catalysis was still carried out in a controlled manner and the temperature of the resin system was also kept stable.

It could be seen from the above experimental data that under the action of these free radicals, the isocyanate group would undergo a slow and controllable trimerization reaction, and the isocyanate group was gradually consumed to form a new isocyanurate group.

While the present invention has been described with its preferable embodiments as above, such embodiments are not intended to limit the present invention. It is obvious to those skilled in the art that various changes and modifications can be made without departing from the spirit and scope of the present invention. The protection scope of the present invention should be determined by the scope of the claims of the present patent application.

## Claims

1.  A process for preparing a (poly)isocyanurate, which is produced by reaction of a reaction system comprising the following components:

    A) one or more polyisocyanates;
    B) at least one free radical initiator.

2.  The process for preparing a (poly)isocyanurate according to Claim 1, which is **characterized in that**, said isocyanate is selected from a group consisting of an aromatic isocyanate, preferably diphenylmethane diisocyanate, toluene diisocyanate, diphenylmethane diisocyanate or a combination thereof, more preferably diphenylmethane diisocyanate or (poly)diphenylmethane diisocyanate or a combination thereof.

3.  The process for preparing a (poly)isocyanurate according to Claim 1 or 2, which is **characterized in that**, the content of said free radical initiator is 0.1-5 pbw, preferably 0.2-4 pbw, more preferably 0.4-3 pbw, based on the total weight of said reaction system.

4.  The process for preparing a (poly)isocyanurate according to Claim 1 or 2, which is **characterized in that**, said free radical initiator is selected from a group consisting of a peroxide and/or an azo compound.

5.  The process for preparing a (poly)isocyanurate according to Claim 4, which is **characterized in that**, said peroxide is selected from a group consisting of ketone peroxide, carbonate peroxide, acyl peroxide, ester peroxide, hydrogen peroxide, alkyl peroxide or any combination thereof, preferably tert-butyl benzoate peroxide, benzoyl peroxide or a combination thereof.

6. The process for preparing a (poly)isocyanurate according to Claim 4, which is **characterized in that**, said azo compound is selected from a group consisting of azobisisobutyronitrile, azobisisoheptonitrile or any combination thereof.

7. The process for preparing a (poly)isocyanurate according to Claim 1 or 2, which is **characterized in that**, the reaction time of said reaction system is >5 hours, preferably >10 hours, particularly preferably >24 hours.

8. The process for preparing a (poly)isocyanurate according to Claim 1 or 2, which is **characterized in that**, the difference between the temperatures after and before the reaction of said reaction system is $<\pm 2°C$.

9. A (poly)isocyanurate prepared with the process for preparing a (poly)isocyanurate according to any of Claims 1-8.

10. Use of a free radical initiator in the catalysis of a trimerization reaction of an isocyanate to prepare a (poly)isocyanurate.

11. Use according to Claim 10, which is **characterized in that**, the content of said free radical initiator is 0.1-5 pbw, preferably 0.2-4 pbw, more preferably 0.4-3 pbw, based on the total weight of the reaction system of said trimerization reaction.

12. Use according to Claim 10 or 11, which is **characterized in that**, said free radical initiator is selected from a group consisting of a peroxide and/or an azo compound.

13. Use according to Claim 10 or 11, which is **characterized in that**, said peroxide is selected from a group consisting of ketone peroxide, carbonate peroxide, acyl peroxide, ester peroxide, hydrogen peroxide, alkyl peroxide or a combination thereof, preferably tert-butyl benzoate peroxide, benzoyl peroxide or a combination thereof.

14. Use according to Claim 10 or 11, which is **characterized in that**, said azo compound is selected from a group consisting of azobisisobutyronitrile, azobisisoheptonitrile or a combination thereof.

15. Use of the (poly)isocyanurate prepared with the process for preparing a (poly)isocyanurate according to any of Claims 1-8 in the heat-insulation.

16. A reaction system for preparing a polyurethane-(poly)isocyanurate compound, comprising:

   a (poly)isocyanurate prepared with the process for preparing a (poly)isocyanurate according to any of Claims 1-8;
   at least one polyol; and
   optionally, at least one foaming agent.

17. A polyurethane-(poly)isocyanurate compound prepared from the reaction system for preparing a polyurethane-(poly)isocyanurate compound according to Claim 16.

18. A heat-insulation material, comprising a (poly)isocyanurate prepared with the process for preparing a (poly)isocyanurate according to any of Claims 1-8.

19. The heat-insulation material according to Claim 18, which is selected from a group consisting of a thermal insulation board, a refrigerator wall, a cold-insulation tube shell, a refrigerated truck compartment side panel and a car-carried refrigerator wall.

Fig. 1

Europäisches
Patentamt

European
Patent Office

Office européen
des brevets

## EUROPEAN SEARCH REPORT

**Application Number**

EP 19 17 8722

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | EP 0 315 692 A1 (MITSUI TOATSU CHEMICALS [JP]) 17 May 1989 (1989-05-17)<br>* page 10; example 2 *<br>* page 13; example 6 *<br>* page 9, line 4 - line 7 *<br>----- | 9<br><br>1-8,<br>10-19 | INV.<br>C08G18/79<br>C08G18/76<br>C08G18/18<br>C08G18/16<br>C08G18/42 |
| A | EP 2 048 172 A1 (CRAY VALLEY LTD [GB]; CRAY VALLEY SA [FR])<br>15 April 2009 (2009-04-15)<br>* paragraph [0035] *<br>* paragraph [0044] *<br>----- | 1-19 | C08G18/44<br>C08G18/40<br>C08G18/48<br>C07D251/34 |
| X<br>A | US 2011/201706 A1 (ATHEY PHILLIP S [US] ET AL) 18 August 2011 (2011-08-18)<br>* paragraph [0064] *<br>* paragraph [0071]; example 2 *<br>----- | 15-19<br><br>1-14 | ADD.<br>C08G101/00<br>C08G18/02 |

|  |
|---|
| TECHNICAL FIELDS SEARCHED (IPC) |
| C08G<br>C07D |

The present search report has been drawn up for all claims

| Place of search | Date of completion of the search | Examiner |
|---|---|---|
| Munich | 20 September 2019 | Ojea Jimenez, Isaac |

EPO FORM 1503 03.82 (P04C01)

## ANNEX TO THE EUROPEAN SEARCH REPORT
## ON EUROPEAN PATENT APPLICATION NO.

EP 19 17 8722

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2019

| Patent document cited in search report | | Publication date | Patent family member(s) | | Publication date |
|---|---|---|---|---|---|
| EP 0315692 | A1 | 17-05-1989 | BR | 8807066 A | 31-10-1989 |
| | | | EP | 0315692 A1 | 17-05-1989 |
| | | | KR | 897001569 A | 21-12-1989 |
| | | | US | 4937339 A | 26-06-1990 |
| | | | WO | 8809329 A1 | 01-12-1988 |
| EP 2048172 | A1 | 15-04-2009 | AU | 2008310034 A1 | 16-04-2009 |
| | | | BR | PI0817496 A2 | 08-09-2015 |
| | | | CA | 2702074 A1 | 16-04-2009 |
| | | | CN | 101821308 A | 01-09-2010 |
| | | | EA | 201070445 A1 | 30-08-2010 |
| | | | EP | 2048172 A1 | 15-04-2009 |
| | | | EP | 2201056 A1 | 30-06-2010 |
| | | | ES | 2453967 T3 | 09-04-2014 |
| | | | KR | 20100084508 A | 26-07-2010 |
| | | | MX | 340599 B | 18-07-2016 |
| | | | MY | 160144 A | 28-02-2017 |
| | | | US | 2010239847 A1 | 23-09-2010 |
| | | | WO | 2009046902 A1 | 16-04-2009 |
| | | | ZA | 201001923 B | 24-11-2010 |
| US 2011201706 | A1 | 18-08-2011 | CN | 102272183 A | 07-12-2011 |
| | | | EP | 2346916 A2 | 27-07-2011 |
| | | | ES | 2561042 T3 | 24-02-2016 |
| | | | JP | 2012508300 A | 05-04-2012 |
| | | | US | 2011201706 A1 | 18-08-2011 |
| | | | WO | 2010054311 A2 | 14-05-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101848951 A
- CN 108026232 A
- GB 1200942016 T
- GB 1200881992 T